(19) Europäisches Patentamt
European Patent Office
Office européen des brevets

(11) EP 0 388 810 B1

(12) EUROPEAN PATENT SPECIFICATION

(45) Date of publication and mention of the grant of the patent:
**19.06.1996 Bulletin 1996/25**

(51) Int Cl.[6]: **C11D 1/66**, C11D 3/20, A61K 7/00, A61K 7/28, A61K 7/075

(21) Application number: **90104998.1**

(22) Date of filing: **16.03.1990**

(54) **Neutral liquid detergent composition**

Neutrale Flüssigwaschmittelzusammensetzung

Composition détergente liquide et neutre

(84) Designated Contracting States:
**DE ES FR GB**

(30) Priority: **20.03.1989 JP 68698/89**

(43) Date of publication of application:
**26.09.1990 Bulletin 1990/39**

(73) Proprietor: **KAO CORPORATION**
**Chuo-ku Tokyo (JP)**

(72) Inventors:
- **Deguchi, Katsuhiko**
  **Utsunomiya-shi, Tochigi (JP)**
- **Saito, Kozo**
  **Utsunomiya-shi, Tochigi (JP)**
- **Saijo, Hiroyuki**
  **Utsunomiya-shi, Tochigi (JP)**

(74) Representative:
**Wächtershäuser, Günter, Prof. Dr.**
**Patentanwalt,**
**Tal 29**
**80331 München (DE)**

(56) References cited:
**EP-A- 0 070 077**
**EP-A- 0 105 556**
**US-A- 3 721 633**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

**Description**

BACKGROUND OF THE INVENTION

Field of the Invention:

The present invention relates to a detergent composition, and, more particularly, to a neutral liquid detergent composition exhibiting remarkably reduced irritation and damage to the hair or the skin, possessing high foaming capability, and imparting a favorable feeling upon use. After washing, the detergent components can easily be rinsed off leaving the least amount of water on the washed object such as tableware.

Neutral liquid compositions comprising alkyl glycoside and ammonium xylene sulfonate are known from EP-A-070 077. Other liquid compositions comprising glycoside are known from EP-A-105 556.

Description of the Background Art:

Because of increased awareness towards safety of the human body in recent years, a number of trials have been undertaken in order to moderate the action of detergent compositions which frequently contact the skin, such as laundry detergents, dishwashing detergents, household detergents, hair shampoos, body shampoos, and the like. One of the approaches is adjusting the pH of detergent compositions to a weakly acidic range, e.g. pH 5-6, close to the pH of human skins so as to be mild to the skin. Another approach is using a low irritant detergent as a major detergent base component. Amino acid surfactants, alkyl phosphate surfactants, or the like are used as a low irritant detergent (JP-A-50-40125, JP-A-51-426023, JP-A-55-9033 and JP-A-58-27319).

Even though these surfactants are low irritant, they do not necessarily exhibit sufficient detergency and foaming capability when used alone. Poor water-solubilty is also their drawback. Sodium alkylbenzenesulfonates have conventionally been popular detergent base components for dishwashing detergents because of their strong detergency. The compounds, however, give a strong defattying action to the skin and thus can be a cause of roughened hands.

Because of these reasons, sodium alkylethoxysulfates which are less irritant to the skin have gained a wider popularity as the base components for dishwashing detergents in recent years. Their detergent performances are even promoted when they are used together with adjuvants such as ternary amine oxides, higher fatty acid diethanolamides, and the like. Their action to the skin is also reduced to some extent by such a combination as compared with conventional detergent composition.

The degree of mildness which has heretofore been achieved by the above-mentioned various detergent compositions are not sufficient.

Alkyl glycosides, which are saccharide-derived surface active agents, are less irritant compounds. Although they are non-ionic surfactants, they not only produce stable foam themselves but also can act as a foam stabilizer when used together with anionic surfactants. Thus, alkyl glycosides are gaining much attention in recent years.

JP-A-58-104625, for example, discloses a foaming surfactant composition comprising an alkyl glycoside and nonionic surface active agent, and JP-A-62-74999 discloses a liquid dishwashing detergent composition comprising an alkyl glycoside, nonionic surface active agent, and a fatty acid alkanolamide. They claim that the composition is low irritant and has an excellent detergency. Even though these detergent compositions exhibit performances better than conventional detergent compositions containing polyoxyethylenealkylethers as a base component, such performances are still to be improved. They have problems such as an inferior feeling upon use, poor rinsability of the foam, and insufficient drainability.

In view of this situation, the present inventors have conducted extensive studies to make the maximum use of the characteristics of alkyl glycosides, and found that a detergent composition exhibiting an excellent feeling upon use, good rinsability of the foam, and superior drainability by using alkyl glycosides together with a specific amount of certain types of higher alcohol and water-soluble salt. Such a finding has led to the completion of this invention.

SUMMARY OF THE INVENTION

Accordingly, an object of the present invention is to provide a neutral liquid detergent composition according to Claim 1.

Other objects, features and advantages of the invention will hereinafter become more readily apparent from the following description.

DETAILED DESCRIPTION OF THE INVENTION AND PREFERRED EMBODIMENTS

Desirable alkyl glycosides used in the composition of the present invention are those represented by following

formula (I),

$$R_1(OR_2)_xG_y \qquad (I)$$

wherein $R_1$ is a linear or branched alkyl, alkenyl, or alkylphenyl group having 8-18 carbon atoms, wherein $R_2$ is an alkylene group having 2-4 carbon atoms, G is a reduced saccharide residue having 5-6 carbon atoms, x is a mean value of 0-5, and y is a mean value of 1.2-1.42.

Among the defined mean value of x, 0-5, especially preferable range is 0-2. This value affects the water-solubility and crystalline property of the alkyl glycosides. Higher the value x, are the alkyl glycosides more soluble in water and less crystalline.

The saccharide chain bonds may be 1-2, 1-3, 1-4, 1-6, α-pyranoside, β-pyranoside, or franoside, or a mixture thereof. The value y is 1.2-1.42. The value y is determined by the proton NMR method. $R_1$ in formula (I) is a $C_{8-18}$ linear or branched alkyl, alkenyl, or alkylphenyl group. From the aspect of water-solubility and foaming capability, a preferable carbon number is $C_{10-14}$. $R_2$ in formula (I) is a $C_{2-4}$ alkylene group. From the aspect of water-solubility and the like, a preferable carbon number is $C_{2-3}$. The structure of G is a reducing saccharide residue having 5 to 6 carbon atoms. Monosaccharides include glucose, galactose, xylose, mannose, lyxose, arabinose, and the like, as well as their mixtures. Among these, from the aspect of easy availability and low cost, preferable monosaccharide is glucose.

The composition of the present invention contains 3-60% by weight of component (a).

Component (b) of the present invention is higher alcohols having a linear or branched alkyl group, of which the average carbon atom content is 8-14. Higher alcohols having the average carbon atom content outside this range are not preferable, since they cannot sufficiently exhibit the intended effects of the present invention in conjunction of component (c).

A preferable amount of component (b) in the composition is 0.001-0.5% by weight, with the especially preferable range being 0.005-0.1% by weight. The amount of component (b) exceeding 0.5% by weight does not not bring about the intended effects. In some cases, the odor of the resulting composition is so impaired that its commercial value is lost.

Component (c) is selected from one or more of water-soluble organic or inorganic salts mentioned above. Among them, especially preferable salts are sodium and potassium sulfates, chlorides, borates, phosphates, p-toluensulfonates, benzoates, citrates, and edates.

Component (c) is incorporated into the composition of the present invention in an amount of 0.01-4% by weight.

Furthermore, it is desirable that the ratio by weight of component (b)/(c) be in a range of 1/5,000-50/1, and preferably of 1/3,000-30/1.

Besides the above essential components, commonly known surfactant detergents can optionally be incorporated into the composition of the present invention in order to promote its detergency and foaming capability. Such surfactants may include, for example, nonionic surfactants such as polyoxyethylenealkylethers (EO=4-20, alkyl group: $C_{7-18}$, linear or branched), $C_{3-22}$ higher fatty acid mono- or dialkanolamides (alkanol group: $C_{2-3}$), tert-alkylamine oxides (alkyl group: $C_{8-18}$, linear or branched), and the like; anionic surfactants such as $C_{8-20}$ α-olefin sulfonates (Na, K, Mg, triethanolamine (TEA), $NH_4$ salts), polyoxyethylenealkyl sulfates (EO=2-8; alkyl group: $C_{8-18}$, linear or branched; Na, K, Mg, TEA, $NH_4$ salts), salts of α-sulfo-fatty acid ester represented by the formula,

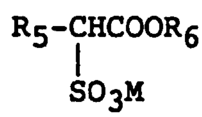

(wherein $R_5$: $C_{8-18}$, $R_6$: $C_{1-4}$, M: alkali metal), N-acylglutamates (acyl group: $C_{8-18}$; Na, K, TEA salts), monoalkyl phosphates (alkyl group : $C_{8-18}$; Na, K, TEA, arginine salts), linear alkylbenzenesulfonates (alkyl group: $C_{10-18}$; Na, K, Mg salts), and the like; and amphoteric surfactants such as alkyl betaine, alkyl sulfobetaine, alkyl hydroxysulfobetaine, and the like.

In detergent compositions of the present invention, component (a), alkyl glycoside, is used as a base component together with 1-20% by weight of the above-mentioned anionic or amphoteric surfactants and 1-10% by weight of nonionic surfactants other than alkyl glycosides, remarkably improved detergency can be exhibited without impairing their foaming capability.

The pH range of a raw liquid of the detergent composition of the present invention is preferably 6-8, and especially preferably 6.5-7.5.

In addition to the above components, other optional components can be formulated to the composition of the present invention to the extent that its stability, detergency, and foaming capability are not adversely affected. Such optional components include lower aliphatic alcohols, e.g. ethanol; solubilizers, e.g. urea; viscosity adjusting agents, e.g. clay minerals, water-soluble polymers; water-insoluble abrasives, e.g. calcite, silica, calcium phosphate, zeolite, polyethylene, nylon, polystyrene; moisturizing agents, e.g. glycerol, sorbitol; feeling improvers, e.g. cationized cellulose; enzyme; perfumes; pigments; antiseptics; antifungal agents; and the like.

The detergent composition of the present invention thus prepared has excellent foaming capability, is mild to the skin and the hair, and imparts a favorable feeling to the hands during and after use. The foam is easily rinsed off and after washing water is easily drained from the tableware and the like. Thus, it is a neutral detergent composition having a high practical value.

Other features of the invention will become apparent in the course of the following description of the exemplary embodiments which are given for illustration of the invention and are not intended to be limiting thereof.

## EXAMPLES

### Example 1

The detergent compositions having the formulations shown in Table 1 were prepared. Their foaming capability, rinsability, feeling to the hands during and after use, and drainability were evaluated. The results are shown in Table 1.

The feeling to the hands during and after use was tested using composition A (Composition 11 in Table 1) as a standard. Evaluation was made by comparing the feelings given to the hands when composition A and composition B (Compositions 1-10 in Table 1) were used.

<Test Methods and Evaluation Standards>

(1) Foaming capability

Foaming capability was measured on samples prepared by adding a commercially available butter in an amount of 0.1%, as a dirt component, to 0.5% detergent composition solutions. Forty milliliters of the solution of the detergent composition to which butter was added was charged into a glass cylinder and subjected to a rotational stirring for 10 minutes. The height of the foam produced was measured after termination of the stirring.

(2) Rinsability

Three liters of a sample solution with 0.25% detergent concentration was charged into a container (diameter: 30 cm, height: 12 cm) and rotationally stirred for 10 minutes, after which the liquid was discharged through an opening at the bottom of the container, leaving the foam in the container. Three liters of tap water was then charged to the container, and the ten minutes stirring and discharge of liquid were repeated. This procedure was repeated until no foam was observed in the container. The number of times of tap water charge and discharge required for the foam to diminish was taken as the standard of rinsability.

(3) Feeling to the hands during and after use

Two 10% detergent composition solutions, A and B, at 40°C were charged into 2 1 beakers. Feeling of solutions A and B to the hands was sensually evaluated according to the following standard.

(i) Feeling of the solution

Hands were dipped into solutions A and B, i.e., one hand to solution A and the other to solution B. After 10 minutes, the feeling to the hands was compared according to the following standard.

| | |
|---|---|
| B is less slippery than A | +2 |
| B is slightly less slippery than A | +1 |
| Cannot tell which is more slippery | 0 |
| B is slightly more slippery than A | -1 |
| B is more slippery than A | -2 |

(ii) Feeling after use

After the above test, the hands were thoroughly washed with water and wiped out with a dry towel. Then feeling of the hands were compared according to the following standard.

| | |
|---|---|
| B is less sticky than A | +2 |
| B is slightly less sticky than A | +1 |
| Cannot tell which is more sticky | 0 |
| B is slightly more sticky than A | -1 |
| B is more sticky than A | -2 |

The above tests were carried out by 10 panelists. The total scores gained by solution B was taken as the feeling of the solution B.

(4) Drainability

Three liters of a 0.15 % detergent composition solution (in 3.5°DH at 25°C) was charged into a container (diameter: 30 cm, depth: 12 cm). A piece of porcelain dish having a 25 cm diameter was dipped into the solution for 3 minutes. The dish was taken out from the solution and dried at 25°C. This procedure was repeated 3 times. After this, the dish was again dipped into the solution, following which it was taken out from the solution and rinsed for 30 seconds in a water stream and dried. The number of water spots remaining on the surface of the dish was counted to evaluate the drainability according to the following standard.

Evaluation Standard:

A: No water spots are present
B: Less than 20 water spots are present
C: 20 or more of water spots are present

TABLE 1

| Component | | Example Compositions | | | | | | Comparative Compositions | | | | |
|---|---|---|---|---|---|---|---|---|---|---|---|---|
| | | 1 | 2 | 3 | 4 | 5 | 6 | 7 | 8 | 9 | 10 | 11 |
| Component (a) | | | | | | | | | | | | |
| Alkyl glycoside: in formula (I), x=0, G=glucose residue, | | | | | | | | | | | | |
| $R_1$: $C_{9-11}$ alkyl, y=1.2 | | 23 | 23 | - | - | 23 | - | 23 | 23 | - | - | 23 |
| $R_1$: $C_{9-11}$ alkyl, y=1.42 | | - | - | 23 | 23 | - | 23 | - | - | 23 | 23 | - |
| Component (b) | Average C | | | | | | | | | | | |
| n-Hexyl alcohol | 6.0 | - | - | - | - | - | - | 0.1 | - | - | - | - |
| n-Octyl alcohol | 8.0 | 0.1 | - | - | - | 0.05 | - | - | - | 0.7 | - | - |
| n-Dodecyl alcohol | 12.0 | - | 0.1 | - | - | 0.05 | - | - | - | - | - | - |
| Dobanol 23 1) | 12.0 | - | - | 0.1 | - | - | 0.1 | - | - | - | 0.1 | 0.1 |
| Alfol 2) | 12.9 | - | - | - | 0.1 | - | - | - | - | - | - | - |
| Dobanol 45 3) | 14.3 | - | - | - | - | - | - | - | 0.1 | - | - | - |
| Component (c) | | | | | | | | | | | | |
| $Na_2SO_4$ | | 1.0 | - | - | - | - | 0.5 | 1.0 | - | - | - | - |
| KCl | | - | 1.0 | - | - | - | - | - | - | 1.0 | - | - |
| $NaH_2PO_4$ | | - | - | - | - | - | 0.1 | - | - | - | - | - |
| Sodium malate | | - | - | 1.0 | - | - | - | - | 1.0 | - | - | - |

TABLE 1 (Continued)

| | Example Compositions | | | | | | Comparative Compositions | | | | |
|---|---|---|---|---|---|---|---|---|---|---|---|
| Component | 1 | 2 | 3 | 4 | 5 | 6 | 7 | 8 | 9 | 10 | 11 |
| Sodium lactate | - | - | - | 1.0 | - | - | - | - | - | - | - |
| Sodium p-toluene sulfonic acid | - | - | - | - | 0.5 | 0.4 | - | - | - | 1.0 | - |
| Disodium edate | - | - | - | - | 0.5 | - | - | - | - | - | - |
| Water | Balance | Balance | Balance | Balance | Balance | Balance | Balance | Balance | Balance | Balance | Balance |
| pH 4) (raw liquid) | 7.0 | 6.5 | 7.3 | 6.2 | 7.9 | 7.1 | 7.0 | 7.5 | 6.6 | 6.8 | 7.0 |
| Evaluation | | | | | | | | | | | |
| Foaming capability (mm) | 90 | 89 | 92 | 91 | 92 | 92 | 85 | 83 | 82 | 84 | 85 |
| Rinsability (times) | 5 | 5 | 5 | 5 | 5 | 5 | 7 | 7 | 8 | 8 | 7 |
| Felling to the hands | | | | | | | | | | | |
| During use | +8 | +8 | +9 | +8 | +8 | +8 | +2.5 | +2.1 | +1.6 | +0.5 | ±0 |
| After use | +8 | +8 | +9 | +8 | +8 | +8 | +2.5 | +2.0 | +1.6 | +0.6 | ±0 |
| Drainability | A | A | A | A | A | A | B | C | C | C | C |

1) Synthetic alcohol, product of Shell Co., alkyl group composition: $C_{12}/C_{13}/C_{14}$=36/63/1, liner/branched=82/18

2) Synthetic alcohol, product of Conoco Co., alkyl group composition: $C_{12}/C_{14}/C_{16}$=54/45/1, liner/branched=97/3

3) Synthetic alcohol, product of Shell Co., alkyl group composition: $C_{12}/C_{13}/C_{14}/C_{15}$=1/1/62/36, liner/branched=79/21

4) pH was appropriately adjusted with $H_2SO_4$ or NaOH.

Example 2

Light duty liquid detergent composition for laundry

| | % by weight |
|---|---|
| Alkyl glycoside 1) | 20 |
| Sodium polyoxyethylene ($\bar{p}$=2.5)-alkyl($C_{12-13}$)ether sulfate | 10 |
| Softhanol 70 2) | 5 |
| Dobanol 23 3) | 0.1 |
| $NaBO_2$ | 0.1 |
| Sodium citrate | 3 |
| Ethanol | 4 |
| Perfume, enzyme | Small amount |
| Water | Balance |
| | 100 |
| (The above mixture was adjusted to pH 8.0) | |

1) In formula (I), x=1, y=1.35, $R_1$=$C_{10-12}$ alkyl, $R_2$=$C_2$ alkylene, G=glucose residue

2) Trademark, a nonionic surfactant produced by Nippon Shokubai Kagakukogyo Co., Ltd.

3) Trademark, synthetic alcohol produced by Shell Co., Ltd.

Example 3

Dishwasing detergent composition

| | % by weight |
|---|---|
| Alkyl glycoside 1) | 20 |

| | |
|---|---|
| Sodium polyoxyethylene($\bar{p}$=4)-laurylether sulfate | 5 |
| Perm oil fatty acid diethanolamide | 2 |
| Decyl alcohol | 0.05 |
| Tetradecyldimethylamine oxide | 2 |
| Sodium sulfate | 2.5 |
| Ethanol | 3 |
| Perfume, enzyme | Small amount |
| Water | Balance |
| | 100 |

(The above mixture was adjusted to pH 7.0)

1) In formula (I), x=0, y=1.3, $R_1=C_{9-11}$ alkyl, G=glucose residue

Example 4

Bath cleanser

| | % by weight |
|---|---|
| Alkyl glycoside 1) | 4 |
| Sodium linear alkyl($C_{12-13}$)-benzenesulfonate | 2 |
| Emulgen 120 2) | 1 |
| Alfol 1214 3) | 0.5 |
| Sodium benzoate | 1.0 |
| Dipotassium edate | 0.1 |
| Perfume, enzyme | Small amount |
| Water | Balance |
| | 100 |
| (The above mixture was adjusted to pH 6.3) | |

1) In formula (I), x=0, y=1.4, $R_1=C_{8-12}$ alkyl, G=glucose residue
2) Trademark, a nonionic surfactant produced by Kao Corp.
3) Trademark, synthetic alcohol produced by Conoco Co.

Obviously, numerous modifications and variations of the present invention are possible in light of the above teachings. It is therefore to be understood that within the scope of the appended claims, the invention may be practiced otherwise than as specifically described herein.

## Claims

1. A neutral liquid detergent composition comprising:

(a) 3 to 60 % by weight of an alkyl glycoside represented by formula (I) as a non-ionic surfactant

$$R_1(OR_2)_xG_y \quad (I)$$

wherein $R_1$ is a linear or branched alkyl, alkenyl, or alkylphenyl group having 8 to 18 carbon atoms, wherein $R_2$ is an alkylene group having 2 to 4 carbon atoms, G is a reducing saccharide residue having 5 to 6 carbon atoms, x is a mean value of 0 to 5, and y is a mean value of 1.2 to 1.42;
(b) 0.001 to 0.5 % by weight of a higher alcohol having 8 to 14 carbon atoms; and
(c) 0.01 to 4 % by weight of at least one water-soluble organic or inorganic salt selected from the group consisting of sulfates, chlorides, borates, phosphates, p-toluensulfonates, m-xylenesulfonates, benzoates, malates, succinates, tartarates, citrates, lactates, and edates of sodium and potassium.

2. A neutral liquid detergent composition according to Claim 1, further comprising 1 to 20 % by weight of one or more surfactants, selected from anionic surfactants and amphoteric surfactants, and 1 to 10 % by weight of one or more non-ionic surfactants other than said alkyl glycoside.

## Patentansprüche

1. Neutrales Flüssigwaschmittel, umfassend:

(a) 3 bis 60 Gew.-% eines Alkylglycosids, wiedergegeben durch die Formel (I) als nichtionisches Tensid

$$R_1(OR_2)_xG_y \quad (I)$$

worin $R_1$ eine lineare oder verzweigte Alkyl-, Alkenyl- oder Alkylphenylgruppe mit 8 bis 18 Kohlenstoffatomen bedeutet, wobei $R_2$ eine Alkylengruppe mit 2 bis 4 Kohlenstoffatomen darstellt, G einen reduzierenden Saccharidrest mit 5 bis 6 Kohlenstoffatomen bedeutet, x ein Mittelwert von 0 bis 5 ist und y ein Mittelwert von 1,2 bis 1,42 ist;
(b) 0,001 bis 0,5 Gew.-% eines höheren Alkohols mit 8 bis 14 Kohlenstoffatomen und
(c) 0,01 bis 4 Gew.-% mindestens eines wasserlöslichen organischen oder anorganischen Salzes, ausgewählt aus der Gruppe, bestehend aus Sulfaten, Chloriden, Boraten, Phosphaten, p-Toluolsulfonaten, m-Xylolsulfonaten, Benzoaten, Malaten, Succinaten, Tartraten, Citraten, Lactaten und Edaten von Natrium und Kalium.

2. Neutrales Flüssigwaschmittel nach Anspruch 1, das außerdem 1 bis 20 Gew.-% eines oder mehrerer Tenside, ausgewählt aus anionischen Tensiden und amphoteren Tensiden und 1 bis 10 Gew.-% eines oder mehrerer nichtionischer Tenside, die von dem Alkylglycosid verschieden sind, umfaßt.

## Revendications

1. Composition détergente liquide neutre comprenant :

(a) 3 à 60 % en poids d'un alkyl glycoside représenté par la formule (I) en tant qu'agent tensioactif non ionique :

$$R_1(OR_2)_XG_Y \quad (I)$$

dans laquelle $R_1$ est un groupe alkyle, alcényle ou alkylphényle, linéaire ou ramifié, comportant 8 à 18 atomes de carbone, $R_2$ est un groupe alkylène ayant 2 à 4 atomes de carbone, G est un reste de saccharide réducteur ayant 5 à 6 atomes de carbone, x est une valeur moyenne de O à 5 et y est une valeur moyenne de 1,2 à 1,42 ;
(b) 0,001 à 0,5 % en poids d'un alcool supérieur ayant 8 à 14 atomes de carbone ; et
(c) 0,01 à 4 % en poids d'au moins un sel organique ou inorganique, soluble dans l'eau, choisi dans le groupe constitué par les sulfates, les chlorures, les borates, les phosphates, les p-toluènesulfonates, les m-xylènesulfonates, les benzoates, les malates, les succinates, les tartrates, les citrates, les lactates et les édates de sodium et de potassium.

2. Composition détergente liquide neutre selon la revendication 1, comprenant en outre 1 à 20 % en poids d'un ou plus agents tensioactifs choisi parmi les agents tensioactifs anioniques et les agents tensioactifs amphotères et 1 à 10 % en poids d'un ou plusieurs agent tensioactifs non-ioniques autres que ledit alkyl saccharide.